# EUROPEAN PATENT APPLICATION

(11) **EP 4 403 151 A1**
(43) Date of publication of application: **24.07.2024**
(21) Application number: 22910746.1
(22) Date of filing: 25.11.2022
(51) Int. Cl.: A61F 13/51, A61F 13/53, A61F 13/534, A61F 13/539

(54) **ABSORBENT ARTICLE**

(30) Priority: 20.12.2021 JP 2021206503
(71) Applicant: Unicharm Corporation, Ehime 799-0111 (JP)
(72) Inventor: BANDOU, Takeshi, Kanonji-shi, Kagawa 769-1602 (JP); MURAI, Takamasa, Kanonji-shi, Kagawa 769-1602 (JP); WATANABE, Sakiko, Kanonji-shi, Kagawa 769-1602 (JP); MIYAZAKI, Hirokazu, Kanonji-shi, Kagawa 769-1602 (JP); TODA, Haruki, Kanonji-shi, Kagawa 769-1602 (JP)
(74) Representative: Dolleymores
(86) International application number: PCT/JP2022/043501
(87) International publication number: WO 2023/120028

(57) **Abstract**

The present invention provides an absorbent article (1, 100) which is provided with an absorbent core (33a, 33b, 103) and a core wrap sheet (38, 39, 108, 109) that contains recycled pulp which is obtained by recycling disposable absorbent articles, wherein the core wrap sheet (38, 39, 108, 109) is bonded with another separate sheet (34, 35, 104, 105), which is adjacent to the core wrap sheet in the thickness direction of the absorbent article (1, 100), thereby constituting a composite sheet (C1, C2, C3, C4) so that the core wrap sheet is not broken in both of the case where the absorbent article (1, 100) is immersed in physiological saline of 0.9% for 30 minutes, and is subsequently pulled out therefrom and hung for 10 minutes, while being folded in two at the center in the longitudinal direction of the product and the case where the absorbent article (1, 100) is immersed in physiological saline of 0.9% for 30 minutes, and is subsequently pulled out therefrom and hung for 10 minutes, while being folded in two at the center in the width direction of the product.

## Description

### FIELD

The present invention relates to an absorbent article.

### BACKGROUND

In recent years, there is a demand for efforts to achieve the Sustainable Development Goals (SDGs), and in response to this, efforts are being made to utilize resources effectively. For example, a technology is known for collecting pulp fibers and superabsorbent resin (superabsorbent polymer) from used absorbent articles and recycling them. In addition, of such material recycling, there is a demand to promote horizontal recycling, for example, in which pulp, which is the raw material, is collected from used diapers and is utilized to produce diapers again, as an example. For example, Patent Document 1 discloses a paper composition containing pulp and absorbent resin that have been collected from absorbent articles, and discloses recycled paper.

### [CITATION LIST]

### [PATENT LITERATURE]

[Patent Document 1] Japanese Patent Application Publication No. 2021-75819

### SUMMARY

### [TECHNICAL PROBLEM]

The recycled paper described in Patent Document 1 has increased strength by containing an absorbent resin molecular weight of which is reduced, and this makes it possible to use the recycled paper as absorbent sanitary paper, craft paper, copy paper, and the like for use in disposable diapers, menstrual hygiene products, and the like. However, in the case of using such recycled paper as a material for an absorbent article, it is required to have a material strength to the extent that damage of the material is prevented even when used for a long time. In particular, in a sheet material made from the recycled pulp that is obtained by collecting raw material pulp from used diapers and making it be able to reuse, the fiber length of pulp tends to be short due to a cleaning step in the recycling process, and there are cases where sheet material contain foreign substances. As a result, in the process of forming the recycled pulp into a sheet, the bonds between fibers weakens, and this causes a risk that the material strength is impaired. In the case where such a sheet material containing such recycled pulp is used as a core-wrapping sheet that covers the absorbent core of the absorbent article, there is a risk the core-wrapping sheet break if body fluids or liquids from the excrement and the like infiltrate the core-wrapping sheet for a long period of time during use of the absorbent article.

The present invention was achieved in light of conventional problems such as that described above, an aspect of the present invention is to provide an absorbent article in which a core-wrapping sheet made using recycled pulp is less likely to be damaged even after long-time use.

### [SOLUTION TO PROBLEM]

A main aspect of the present invention of achieving the above-described aspect is an absorbent article including: an absorbent core; a core-wrapping sheet that contains recycled pulp that is obtained by recycling a disposable absorbent article; and a separate sheet that is adjacent to the core-wrapping sheet in a thickness direction of the absorbent article, a composite sheet is formed by joining the core-wrapping sheet with the separate sheet so that the core-wrapping sheet does not break when the absorbent article that has been immersed in 0.9% physiological saline solution for 30 minutes is hanged for 10 minutes in any of a case of being folded one time at a center in a product lengthwise direction and a case of being folded one time at a center in a product width direction.
Features of the present invention other than the above will become clear by reading the description of the present specification with reference to the accompanying drawings.

### [ADVANTAGEOUS EFFECT OF THE INVENTION]

According to the present invention, it is possible to provide an absorbent article in which a core-wrapping sheet made using recycled pulp is less likely to be damaged even after long-time use.

### BRIEF DESCRIPTION OF DRAWINGS

FIG. 1 is a schematic plan view of an absorbent pad 1 in an unfolded and stretched state, which is an absorbent article according to the first embodiment, as viewed from the skin surface side.
FIG. 2 is an exploded perspective view of the main part of the absorbent pad 1 shown in FIG. 1.
FIG. 3 is a cross-sectional view taken along line B-B shown in FIG. 1.
FIG. 4 is a plan view showing an application region T1 of a hot-melt adhesive with which a top-surface sheet 34 and a first core-wrapping sheet 38 of the absorbent pad 1 are joined.
FIG. 5 is a plan view which showing an application region T2 of the hot-melt adhesive with which a top-surface sheet 34 and a first core-wrapping sheet 38 of an absorbent pad 1', which is a modified example.
FIG. 6 is a plan view of a second absorbent core 33b and a second core-wrapping sheet 39 of the absorbent pad 1 that is in the unfolded state.
FIG. 7 is a schematic plan view of a disposable diaper 100 in the unfolded and stretched state, which is an absorbent article according to the second embodiment, as viewed from the skin surface side.
FIG. 8 is a cross-sectional view taken along line A-A shown in FIG. 7.
FIG. 9 is a table showing the evaluation results of the tensile strength of sheets.
FIG. 10 shows an example of a load-elongation diagram in a tensile test of a composite sheet B.
FIG. 11 is a plan view showing the application range of the adhesive which joins a top-surface sheet 104 and a skin-side core-wrapping sheet 108.
FIG. 12 is a plan view showing the application range of the adhesive which joins a non-skin-side core-wrapping sheet 109 and a back-surface sheet 105.
FIG. 13 is a plan view further showing an adhesive with which an absorbent core 103 and a non-skin-side core-wrapping sheet 109 are joined, onto the plan view of FIG. 12.

### DESCRIPTION OF EMBODIMENTS

At least the following matters will be clear with the description of this specification and the attached drawings.

An absorbent article including: an absorbent core; a core-wrapping sheet that contains recycled pulp that is obtained by recycling a disposable absorbent article; and a separate sheet that is adjacent to the core-wrapping sheet in a thickness direction of the absorbent article, a composite sheet is formed by joining the core-wrapping sheet with the separate sheet so that the core-wrapping sheet does not break when the absorbent article that has been immersed in 0.9% physiological saline solution for 30 minutes is hanged for 10 minutes in any of a case of being folded one time at a center in a product lengthwise direction and a case of being folded one time at a center in a product width direction.

According to the absorbent article, the core-wrapping sheet in the composite sheet do not break even under the above-mentioned conditions assuming long-term use. Therefore, by joining the core-wrapping sheet and the separate sheet, it is possible to improve the breakage resistance of the core-wrapping sheet itself in the composite sheet to the extent that it can be used without breaking even after being immersed for such a long time.

In such an absorbent article, it is desirable that the absorbent article has a skin-side composite sheet that is the composite sheet placed on the skin side in the thickness direction of the absorbent core, and a non-skin-side composite sheet that is the composite sheet placed on the non-skin side in the thickness direction of the absorbent core, that the core-wrapping sheet in the skin-side composite sheet is a skin-side core-wrapping sheet that is located adjacent to the absorbent core on a skin side, and that the core-wrapping sheet in the non-skin-side composite sheet is a non-skin-side core-wrapping sheet that is located adjacent to the absorbent core on a non-skin side.

According to the absorbent article, the absorbent core swells when it absorbs liquid, and therefore if the core-wrapping sheet is provided so as to wrap around the absorbent core, the swelling of the absorbent core makes a force be applied to the core-wrapping sheet, causing a risk that the core-wrapping sheet on the side that wraps the absorbent core breaks. By arranging the core-wrapping sheet individually on the skin side and the non-skin side, it is possible to reduce the burden on the core-wrapping sheet and to reduce the risk of breakage.

In such an absorbent article, it is desirable that the absorbent core contains superabsorbent polymer, and that a mass ratio of the superabsorbent polymer in the absorbent core is 28% or less.

According to the absorbent article, in the case where the ratio of the superabsorbent polymer is high, when liquid is absorbed, the superabsorbent polymer is not able to remain in the fiber aggregate of the absorbent core, and leakage may occur. By making the mass ratio to 28% or less, it is possible to reduce the risk of leakage and to make the core-wrapping sheet more difficult to break.

In such an absorbent article, it is desirable that the absorbent article includes a first absorbent core that contains at least a superabsorbent polymer, and a second absorbent core that contains at least a superabsorbent polymer, that a mass ratio of the superabsorbent polymer contained in the first absorbent core is greater than a mass ratio of the superabsorbent polymer contained in the second absorbent core, that a first core-wrapping sheet that is arranged so as not to wrap the first absorbent core and that is the core-wrapping sheet is provided, and that a second core-wrapping sheet that wraps the second absorbent core and that is the core-wrapping sheet is provided.

According to the absorbent article, since the first core-wrapping sheet of the first absorbent core, which has a large amount of superabsorbent polymer, is made into a form that does not wrap the first absorbent core, this reduces the burden on the first core-wrapping sheet of the first absorbent core, which has a large amount of the superabsorbent polymer and thereby is more likely to swell, making it possible to reduce the risk that the core-wrapping sheet break

In such an absorbent article, it is desirable that the core-wrapping sheet contains a thermoplastic resin.

According to the absorbent article, it is possible to reduce a risk that the core-wrapping sheet containing the thermoplastic resin is damaged.

In such an absorbent article, it is desirable that the separate sheet contains a thermoplastic resin, and that the core-wrapping sheet and the separate sheet are thermally-fused.

According to the absorbent article, by thermally-fusing the thermoplastic resin of the core-wrapping sheet and the thermoplastic resin of the separate sheet, it is possible to increase the strength of the core-wrapping sheet in the composite sheet.

In such an absorbent article, it is desirable that the thermoplastic resin of the core-wrapping sheet and the thermoplastic resin of the separate sheet are an identical type of thermoplastic resin.

According to the absorbent article, thermal-fusing of the identical type of the thermoplastic resin makes it possible to increase the strength of the core-wrapping sheet in the composite sheet.

In such an absorbent article, it is desirable that the core-wrapping sheet contains a thermoplastic resin, that at least a part of the core-wrapping sheet has an overlapping region where portions of the core-wrapping sheet overlap each other, in a plan view of the absorbent article that is in the unfolded state, and that, in the overlapping region, the portions of the core-wrapping sheet are thermally-fused to each other.

According to the absorbent article, by thermally-fusing the portions of the core-wrapping sheet, it is possible to increase the strength of the core-wrapping sheet and to further reduce the risk of breakage.

In such an absorbent article, it is desirable that a content of the recycled pulp in the core-wrapping sheet being less than 15 wt%, and that a tensile force per unit width at a timing when the core-wrapping sheet starts to break in the composite sheet if the composite sheet is pulled in a fiber orientation direction of the core-wrapping sheet is higher than a tensile force per unit width at a timing when the separate sheet starts to break if the separate sheet alone is pulled in the fiber orientation direction.

According to the absorbent article, the separate sheet is joined to the core-wrapping sheet in which the content of the recycled pulp is less than 15 wt%, and accordingly the strength of the core-wrapping sheet becomes a strength that the core-wrapping sheet do not break unless there is applied a force greater than the tensile force at the timing when the separate sheet alone starts to break. By improving the strength of the core-wrapping sheet, the risk of breakage can be reduced.

In such an absorbent article, it is desirable that, in the composite sheet, the core-wrapping sheet and the separate sheet are joined with a hot-melt adhesive.

According to the absorbent article, by joining with the hot-melt adhesive, it is possible to make the core-wrapping sheet of the composite sheet further less likely to break even when the core-wrapping sheet continues to be infiltrated with liquid.

In such an absorbent article, it is desirable that the hot-melt adhesive is applied in a planar manner to at least a part.

According to the absorbent article, the strength of the core-wrapping sheet can further increase in portions where the hot-melt adhesive is applied without gaps (solid-applied portion).

In such an absorbent article, it is desirable that the composite sheet has a non-joined region where the core-wrapping sheet and the separate sheet are not joined with the hot-melt adhesive.

According to the absorbent article, if the hot-melt adhesive is applied completely without any gap to the entire surface, there is a risk that the composite sheet will become the composite sheet becomes stiff. However, by having non-joining region, the softness can be maintained and a balance between strength and hardness can be achieved.

In such an absorbent article, it is desirable that at least a part of a joining region where the core-wrapping sheet and the separate sheet are joined with the hot-melt adhesive is provided over an entire range of the core-wrapping sheet in the fiber orientation direction.

According to the absorbent article, the tensile strength is high in the fiber orientation direction, and since the joining region of the hot-melt adhesive exists over the entire range of the core-wrapping sheet in the fiber orientation direction, it can make the core-wrapping sheet further less likely to break.

In such an absorbent article, it is desirable that the separate sheet of the non-skin-side composite sheet is a liquid-impermeable sheet member, and that, in the non-skin-side composite sheet, the non-skin-side core-wrapping sheet and the separate sheet are joined with a hot-melt adhesive.

According to the absorbent article, by joining the liquid-impermeable sheet member (film, etc.) with the hot-melt adhesive, it makes the core-wrapping sheet in the non-skin-side composite sheet further less likely to break, a making it possible to reduce a risk of leakage from the non-skin side.

In such an absorbent article, it is desirable that the separate sheet of the non-skin-side composite sheet is located adjacent to the non-skin-side core-wrapping sheet on the non-skin side, that the non-skin-side composite sheet has a non-joined region where the non-skin-side core-wrapping sheet and the separate sheet are not joined with a hot-melt adhesive, that the absorbent core and the non-skin-side core-wrapping sheet have a core joining region where the absorbent core and the non-skin-side core-wrapping sheet are joined to each other with the hot-melt adhesive, and that the non-joining region has a portion that overlaps the core joining region in a plan view of the absorbent article that is in an unfolded state.

According to the absorbent article, there is a risk that the strength of the non-joining region where the hot-melt adhesive is not applied decreases, but by having a portion that overlaps the core joining region where the absorbent core and the non-skin-side core-wrapping sheet are joined, it is possible to suppress the decrease in strength.

In such an absorbent article, it is desirable that the separate sheet of the skin-side composite sheet is a liquid-permeable sheet member, that the separate sheet of the non-skin-side composite sheet is a liquid-impermeable sheet member, and that a tensile force per unit width at a timing when the core-wrapping sheet starts to break in the non-skin-side composite sheet if the non-skin-side composite sheet is pulled in a fiber orientation direction of the core-wrapping sheet is higher than a tensile force per unit width at a timing when the core-wrapping sheet starts to break in the skin-side composite sheet if the skin-side composite sheet is pulled in the fiber orientation direction of the core-wrapping sheet.

According to the absorbent article, the tensile force at the timing when the core-wrapping sheet starts to break in the composite sheet increases as the tensile force at the timing when the separate sheet to be joined starts to break increases. Since the non-skin-side composite sheet is joined to the liquid-impermeable sheet member (separate sheet), the core-wrapping sheet of the non-skin-side composite sheet has a higher tensile force the timing of starting of breakage than the core-wrapping sheet of the skin-side composite sheet. The core-wrapping sheet that is further less likely to break is placed on the non-skin side of the absorbent core, thereby improving the leakage prevention effect.

### First embodiment

The following describes an absorbent pad 1 that absorbs excrement such as urine and feces by way of example of an absorbent article according to the first embodiment. However, the present invention is not limited to the above, and the absorbent article is also applicable to tape-type disposable diapers for adults or infants, menstrual hygiene products, and the like.

### Basic structure of absorbent pad 1

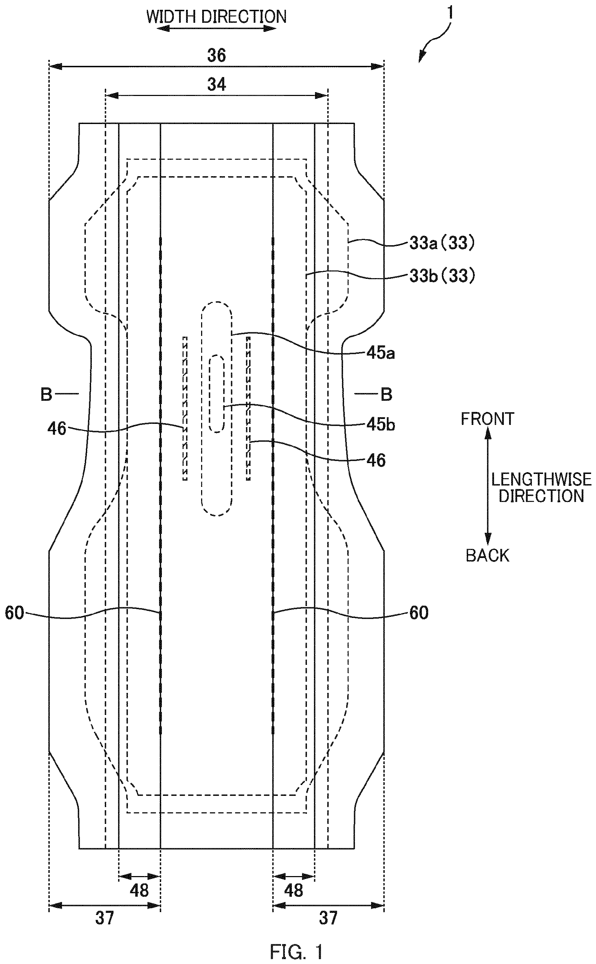

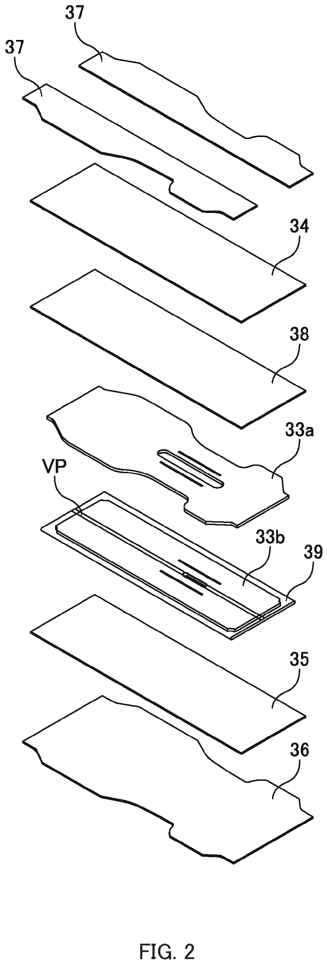

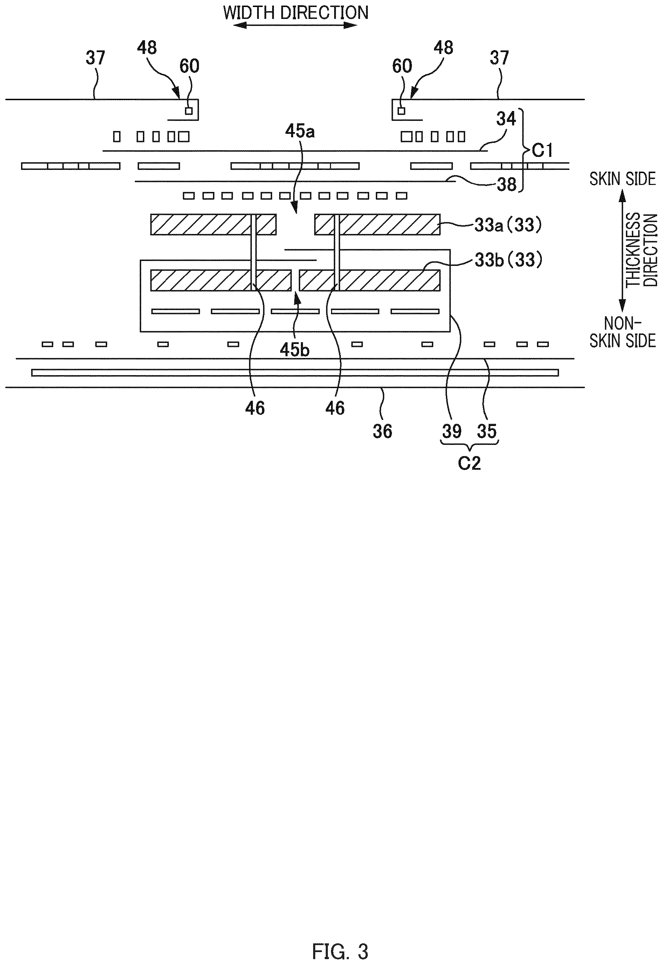

FIG. 1 is a schematic plan view of an absorbent pad 1 in an unfolded and stretched state, which is an absorbent article according to the first embodiment, as viewed from the skin surface side. FIG. 2 is an exploded perspective view of the main part of the absorbent pad 1 shown in FIG. 1. FIG. 3 is a cross-sectional view taken along line B-B shown in FIG. 1.

As shown in FIG. 1, the absorbent pad 1 has a lengthwise direction and a width direction in the unfolded and stretched state. Further, as shown in FIG. 3, the direction in which the constituent members of the absorbent pad 1 are overlaid on each other is referred to as the thickness direction. In the thickness direction, the side that is in contact with the wearer is called the skin side, and the opposite side is called the non-skin side.

Note that the unfolded state of the absorbent pad 1 refers to a state in which the absorbent pad 1 is opened in the lengthwise direction and thus the absorbent pad 1 is unfolded in a plan view. In addition, the "stretched state" of the absorbent pad 1 refers to a state in which the entire absorbent pad 1 (entire product) has been stretched to eliminate wrinkles, or more specifically has been stretched until the dimensions of constituent members of the absorbent pad 1 (e.g., later-described leak-proof-wall elastic members 60, absorbent core 3, and the like) match or are close to the dimensions of the members on their own (in other words, the dimensions in a state where the elastic characteristics of the elastic member are not exhibited).

As shown in FIGS. 1 and 2, the absorbent pad 1 includes a top-surface sheet 34, a back-surface sheet 35, an absorbent body 33, an exterior sheet 36, and a pair of side sheets 37. The top-surface sheet 34 is a liquid-permeable sheet provided on the skin side in the thickness direction with respect to the absorbent body 33, and examples thereof include an air-through nonwoven fabric sheet, a spunbond nonwoven fabric sheet, and the like. The back-surface sheet 35 is a liquid-impermeable sheet provided on the non-skin side in the thickness direction with respect to the absorbent body 33, and examples thereof include a synthetic resin film and a hydrophobic SMS nonwoven fabric sheet and the like. Additionally, the top-surface sheet 34 and the back-surface sheet 35 contain thermoplastic resin (polyethylene (PE), polypropylene (PP), and the like).

The pair of side sheets 37 each are a sheet (e.g., nonwoven fabric sheet, and the like) located on the skin side of the top-surface sheet 34 and extending outward in the width direction from both side portions in the width direction. The exterior sheet 36 is a liquid-impermeable sheet located on the non-skin side in the thickness direction with respect to the back-surface sheet 5, and has a shape (that is, the external shape of the absorbent pad 1) in which the center portion in the lengthwise direction is narrowed inward in the width direction.

The absorbent body 33 is a member that is located between the top-surface sheet 34 and the back-surface sheet 35 in the thickness direction and that has liquid absorbency and liquid retaining properties. In the first embodiment, the absorbent body 33 includes the first absorbent core 33a that is located on the skin side, and the second absorbent core 33b that is located on the non-skin side of the first absorbent core 33a. For example, the first absorbent core 33a and the second absorbent core 33b can be obtained by molding liquid-absorbent fiber such as pulp containing superabsorbent polymer (SAP) into a predetermined shape. Specifically, in the present embodiment, the shape of the first absorbent core 33a is a substantially gourd shape or a substantially hourglass shape, which is elongated in the lengthwise direction and has a portion narrowed inward in the width direction, near the center in the lengthwise direction. On the other hand, the shape of the second absorbent core 33b has a substantially rectangular shape which is long in the lengthwise direction and short in the width direction in a plan view. In the second absorbent core 33b, the dimension in the lengthwise direction is shorter than the dimension of the first absorbent core 33a in the lengthwise direction, and the dimension in the width direction is shorter than the dimension of the first absorbent core 33a in the width direction. Therefore, both edges of the first absorbent core 33a in the lengthwise direction and the width direction are respectively located outside both edges of the second absorbent core 33b in the lengthwise direction and the width direction. Note that there is no particular limitation on the shape of the absorbent body 33.

Further, in the first embodiment, the first core-wrapping sheet 38 is provided on the skin side of the first absorbent core 33a, and a second core-wrapping sheet 39 is provided so as to warp the second absorbent core 33b from the non-skin side. Note that in the first embodiment, the first core-wrapping sheet 38 is arranged so as not to wrap the first absorbent core 33a. The first and second core-wrapping sheets 38 and 39 are made of tissues, for example, and the details thereof will be described later. Further, the first and second core-wrapping sheets 38 and 39 contain thermoplastic resin in addition to pulp fiber as the liquid-absorbent fiber. Examples of the thermoplastic resin include the polyethylene (PE) and the polypropylene (PP).

Further, the absorbent pad 1 has leak-proof walls 48. The leak-proof walls 48 are configured to be able to rise up on the side of the wearer's skin surface, and are provided in pair on the left and right sides. As shown in FIG. 3, the leak-proof walls 48 are each formed as follow: an inner side portion of the side sheet 37 in the width direction is folded back to the non-skin side, and a leak-proof-wall elastic member 60 that stretches and contracts in the lengthwise direction is provided in the folded back portion. The leak-proof wall 48 can suppress the side leakage of excrement. In addition, in FIG. 1, one string-like leak-proof-wall elastic member 60 is arranged spacing on each side in the width direction, but the number of the leak-proof-wall elastic members 60 is not particularly limited.

In the present embodiment, in the absorbent body 33, the first absorbent core 33a has a slit 45a that is recessed, preferably penetrating, in the thickness direction from the skin side towards the non-skin side. The slit 45a is located on the front side with respect to the center of the absorbent pad 1 in the lengthwise direction, and extends in the lengthwise direction through the center of the absorbent body 33 in the width direction. The second absorbent core 33b has a slit 45b that is recessed, preferably penetrating, in the thickness direction from the skin side towards the non-skin side. The slit 45b is located on the front side with respect to the center of the absorbent pad 1 in the lengthwise direction, and extends in the lengthwise direction through the center of the absorbent body 33 in the width direction. The slit 45a and the slit 45b are formed so that at least portions thereof overlap in a plan view. Thereby, when excreted urine reaches the second absorbent core 33b via the slit 45a of the first absorbent core 33a, the urine can be drawn further into the second absorbent core 33b via the slit 45b. The dimensions (length) of the slits 45a and 45b in the lengthwise direction and the dimensions (width) of the slits 45a and 45b in the width direction in a plan view are adjusted as appropriate in consideration of the sizes of the first absorbent core 33a and the second absorbent core 33b. The width and length of the slits 45a and 45b are, for example, 5.0 to 50 mm and 50 to 300 mm.

In the present embodiment, the absorbent body 33 includes a pair of compressed portions 46. The pair of compressed portions 46 extend along the lengthwise direction on both outer sides of the slit 45a in the width direction. The pair of the compressed portions 46 are formed by compressing the first absorbent core 33a and the second absorbent core 33b in the thickness direction from the skin-side surface of the first absorbent core 33a. However, the pair of compressed portions 46 may be formed by compressing both the top-surface sheet 34 and the absorbent body 33. The pair of compressed portions 46 are each formed in a straight-line manner in a plan view, but the shape is not particularly limited. The pair of the compressed portions 46 can prevent urine from moving outward on the top-surface sheet 34 and leaking to the outside, and can make it likely to draw urine into the absorbent body 33.

First core-wrapping sheet 38 and second core-wrapping sheet 39 The first core-wrapping sheet 38 and second core-wrapping sheet 39 in the present embodiment contain recycled pulp that is obtained by recycling used disposable absorbent articles (e.g., used disposable diapers). Generally, in the absorbent article, the material is required to be strong enough to prevent the absorbent core from being exposed to the outside after long periods of use. On the other hand, concerning a sheet containing recycled pulp that is obtained by recovering raw material pulp from collected used disposable absorbent articles and making it be able to reuse by the recycling process, such a sheet has the characteristic that the fiber length of the recycled pulp becomes shorter during a cleaning step. As a result, the strength of the material is impaired, and in the case where, for example, a sheet containing the recycled pulp is used as the core-wrapping sheet, there is a risk the core-wrapping sheet break if body fluids or liquids from the excrement and the like infiltrate the core-wrapping sheet for a long period of time during use of the absorbent article.

In this regard, in the absorbent pad 1, the first core-wrapping sheet 38 (second core-wrapping sheet 39) is joined with a separate sheet adjacent to the first core-wrapping sheet 38 (second core-wrapping sheet 39) in the thickness direction of the absorbent pad 1, to form a composite sheet. Specifically, the separate sheet for the first core-wrapping sheet 38 is the top-surface sheet 34. The first core-wrapping sheet 38 and the top-surface sheet 34 are joined to form the first composite sheet C1. As shown in FIG. 3, the first composite sheet C1 is placed on the skin side of the first absorbent core 33a in the thickness direction. Further, the separate sheet for the second core-wrapping sheet 39 is the back-surface sheet 35, and the non-skin-side surface of the second core-wrapping sheet 39 and the back-surface sheet 35 are joined to form the second composite sheet C2. As shown in FIG. 3, the second composite sheet C2 is placed on the non-skin side of the second absorbent core 33b in the thickness direction.

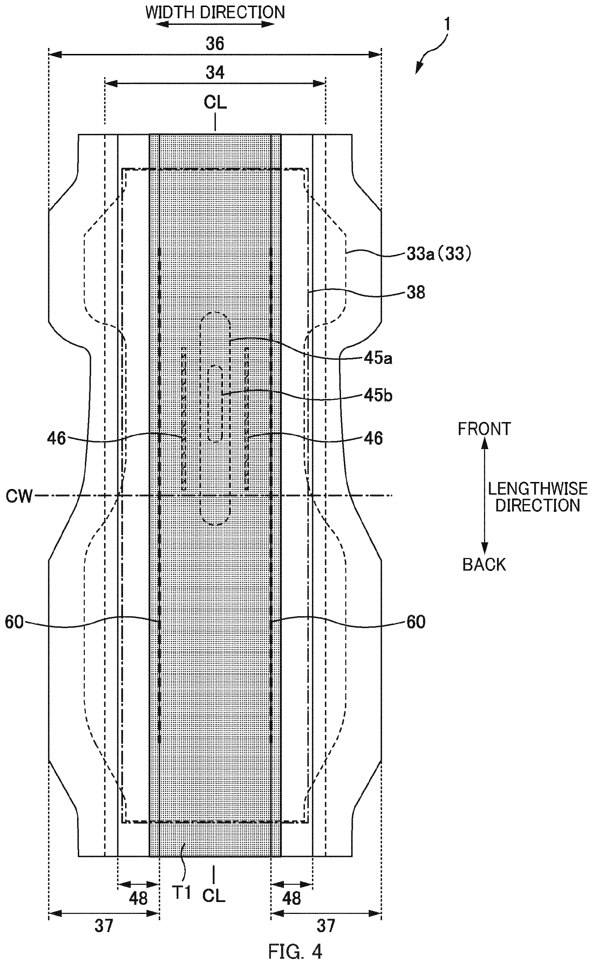

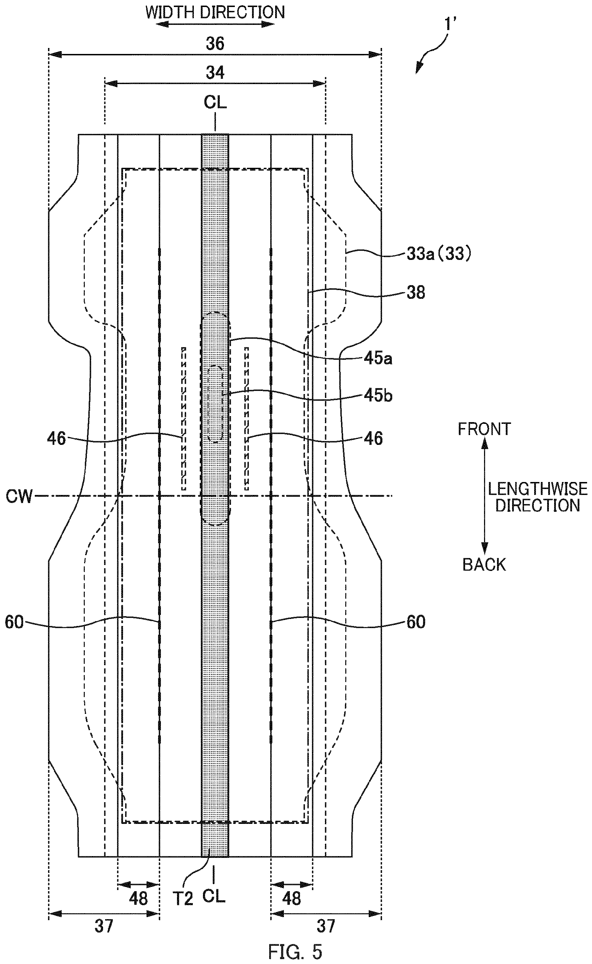

Here, in order to confirm whether the first core-wrapping sheet 38, which contains the recycled pulp, breaks or not even if the absorbent pad 1 is being used for a long time, the following test was conducted. In addition, FIG. 4 is a plan view showing an application region T1 of the hot-melt adhesive with which the top-surface sheet 34 and the first core-wrapping sheet 38 of the absorbent pad 1 are joined, and FIG. 5 is a plan view which showing an application region T2 of the hot-melt adhesive with which a top-surface sheet 34 and a first core-wrapping sheet 38 of the absorbent pad 1', which is a modified example. The absorbent pad 1 and the absorbent pad 1' each have a center line CL that passes through the center in the width direction and that extends in the lengthwise direction, and a center line CW that passes through the center in the lengthwise direction and that extends in the width direction.

### Sample

First, the followings test samples are prepared:
(1) absorbent pad 1 of the present embodiment (a sample where the top-surface sheet 34 and the first core-wrapping sheet 38 are joined in the application region T1 shown in FIG. 4); and
(2) absorbent pad 1', which is the modified example of the absorbent pad 1 (a sample where the top-surface sheet 34 and the first core-wrapping sheet 38 are joined in application region T2 (center only) shown in FIG. 5). For both samples (1) and (2), the recycled pulp content of the first core-wrapping sheet 38 included in the absorbent pad 1 (1') is 7.5 wt%, and the length of the first core-wrapping sheet 38 in the lengthwise direction is 550 mm , and the length in the width direction is 153 mm. Test method

### Test 1

1. Immerse the sample in 10 L of 0.9% physiological saline solution for 30 minutes
2. Pull up the sample, fold the sample one time at the center in the product lengthwise direction, and hang the sample by placing a rod of 10 mm diameter along the center line of the sample passing through the center in the lengthwise direction (center line CW of FIGS. 4 and 5) and leave it for 10 minutes

### Test 2

### 1. Immerse the sample in 10L of 0.9% physiological saline solution for 30 minutes

Pull up the sample, fold the sample one time at the center in the product width direction, and hang the sample by placing a rod of 10 mm diameter along the center line of the sample passing through the center in the width direction (center line CL of FIGS. 4 and 5) and leave it for 10 minutes

### Result

In Test 1, the first core-wrapping sheets 38 of the samples (1) and (2) did not break. However, in Test 2, the first core-wrapping sheet 38 of the sample (1) did not break, but the first core-wrapping sheet 38 of the sample (2) broke.

From the above test, the following fact has been found: similarly to the absorbent pad 1 of the present embodiment, the first composite sheet C1 is formed by joining the first core-wrapping sheet 38 with the top-surface sheet 34 (separate sheet) that is adjacent to the first core-wrapping sheet 38 in the thickness direction of the absorbent pad 1; and the first core-wrapping sheet 38 does not break when the absorbent pad 1 is sufficiently immersed in physiological saline solution and is hanged for 10 minutes in any of a case of being folded one time at the center in the product lengthwise direction and a case of being folded one time at the center in the product width direction. Therefore, like the present embodiment, by joining the first core-wrapping sheet 38 with the top-surface sheet 34, it is possible to improve the breakage resistance of the first core-wrapping sheet 38 itself in the first composite sheet C1 to the extent that it can be used without breaking even under the above conditions. In addition, in the above test, the breakage resistance of the first core-wrapping sheet 38 in the first composite sheet C1 was improved due to the difference in the application region of the hot-melt adhesive that joins the top-surface sheet 34 and the first core-wrapping sheet 38, but the present invention is not limited to this. For example, it is possible to improve the breakage resistance of the first and second core-wrapping sheets 38 and 39 in the composite sheets (C1, C2) as follow: by increasing the amount of hot-melt adhesive per unit area; by increasing the strength of separate sheets in the first and second composite sheets C1 and C2; by changing the amount of the superabsorbent polymer of the first absorbent core 33a and the second absorbent core 33b; and/or by a combination thereof.

Moreover, it is preferable that the mass ratio of the superabsorbent polymer in the first absorbent core 33a is 28% or less. Here, the mass ratio of the superabsorbent polymer in each of the absorbent cores is the ratio of the mass of the superabsorbent polymer to the total mass of the absorbent core (that is, fiber aggregate mass + mass of superabsorbent polymer). In the case where the ratio of the superabsorbent polymer is high, when liquid is absorbed, the superabsorbent polymer is not able to remain in the fiber aggregate of the first absorbent core 33a, and leakage may occur. However, by making the mass ratio of the superabsorbent polymer in the first absorbent core 33a to 28% or less, it is possible to reduce the risk of leakage and to make the first core-wrapping sheet 38 in the first absorbent core 33a more difficult to break. In addition, the following measures are taken for the mass ratio of the superabsorbent polymer in each absorbent core.

In the present embodiment, the mass ratio of the superabsorbent polymer contained in the first absorbent core 33a is greater than the mass ratio of the superabsorbent polymer contained in the second absorbent core 33b. In other words, when absorbing excreted fluid, the first absorbent core 33a is more likely to swell than the second absorbent core 33b. The first core-wrapping sheet 38 of the first absorbent core 33a is provided so as not to wrap around the first absorbent core 33a, and the second core-wrapping sheet 39 of the second absorbent core 33b is provided so as to wrap around the second absorbent core 33b. Since the first core-wrapping sheet 38 of the first absorbent core 33a, which has a large amount of superabsorbent polymer (SAP), is made into a form that does not wrap around the first absorbent core 33a, this reduces the burden on the first core-wrapping sheet 38 of the first absorbent core 33a, which has a large amount of the superabsorbent polymer and thereby is more likely to swell, making it possible to reduce the risk that the first core-wrapping sheet 38 break.

In addition, in the present embodiment, the first absorbent core 33a is located on the skin side and the second absorbent core 33b is located on the non-skin side of the first absorbent core 33a. But the present invention is not limited to this, and the first absorbent core 33a may be located on the non-skin side and the second absorbent core 33b may be located on the skin side of the first absorbent core 33a. That is, the absorbent pad 1 may be configured to provide the absorbent core, which has a high mass ratio of the superabsorbent polymer, on the non-skin side.

In addition, in the case where the thermoplastic resin is contained in the top-surface sheet 34 and the first core-wrapping sheet 38 as described above, for example, if compressed portions such as the above-described pair of compressed portions 46 are formed on the skin side of the top-surface sheet 34, the thermoplastic resin of the top-surface sheet 34 and the thermoplastic resin of the first core-wrapping sheet 38 are thermally-fused. This makes it possible to more firmly join the top-surface sheet 34 and the first core-wrapping sheet 38. Therefore, the strength of the first core-wrapping sheet 38 in the first composite sheet C1 can be increased.

In the case where the top-surface sheet 34 (separate sheet) and the first core-wrapping sheet 38 are thermally-fused, it is desirable that the thermoplastic resin of the first core-wrapping sheet 38 and the thermoplastic resin of the separate sheet are the same type of the thermoplastic resin. Thermal-fusing of the same type of the thermoplastic resin makes it possible to increase the strength of the first core-wrapping sheet 38 in the first composite sheet C1.

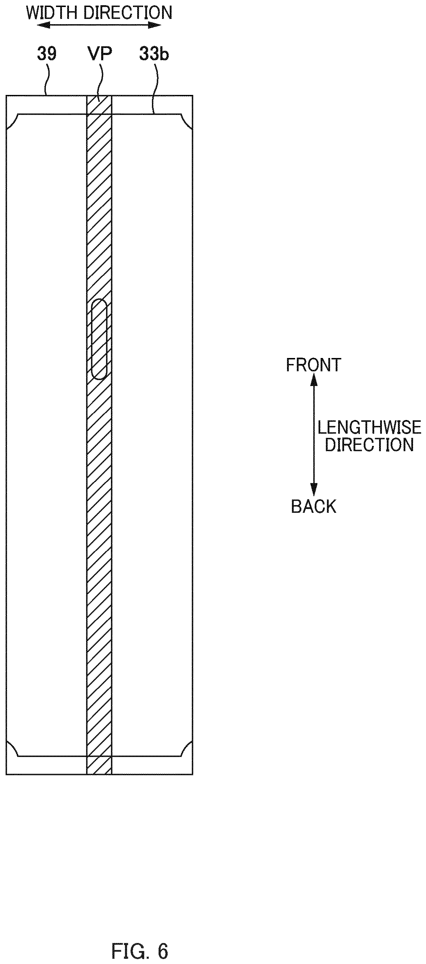

FIG. 6 is a plan view of the second absorbent core 33b and the second core-wrapping sheet 39 of the absorbent pad 1 in the unfolded state. In order to show the overlap of the second core-wrapping sheet 39, only the second absorbent core 33b and the second core-wrapping sheet 39 are shown for convenience. As shown in FIG. 6, at least a part of the second core-wrapping sheet 39 has an overlapping region VP where portions of the second core-wrapping sheet 39 overlap in a plan view. In the overlapping region VP, the portions of the second core-wrapping sheet 39 are thermally-fused to each other. Since the second core-wrapping sheet 39 contains the thermoplastic resin, by thermally-fusing the portions of the second core-wrapping sheet 39, it is possible to increase the strength of the second core-wrapping sheet 39 and to further reduce the risk of breakage.

### Second embodiment

### Basic configuration of diaper 100

The following describes a tape-type disposable diaper for adults (hereinafter also referred to as diaper 100) by way of example of an absorbent article according to the second embodiment.

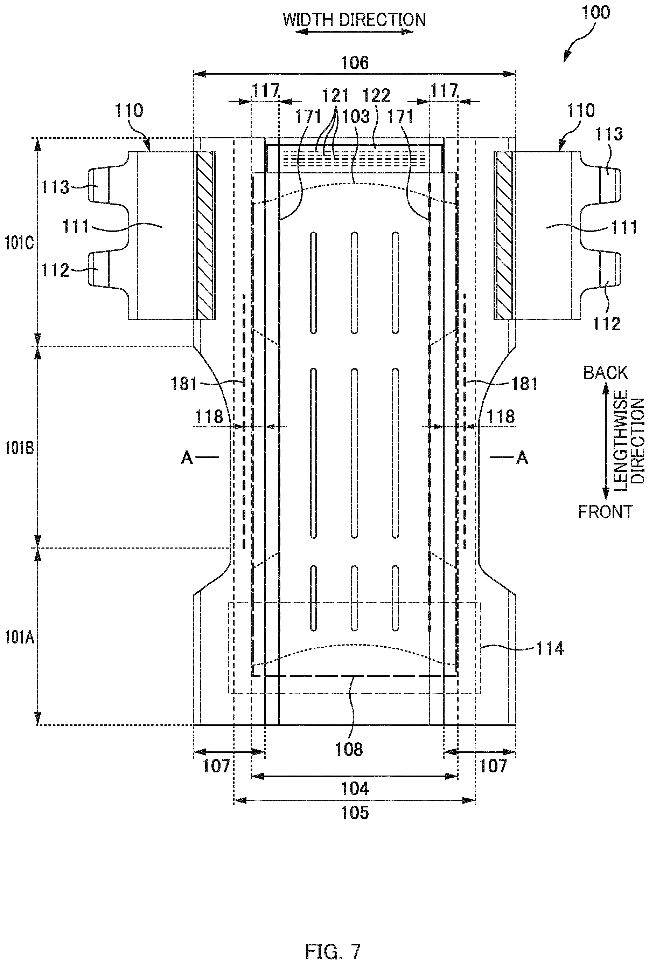

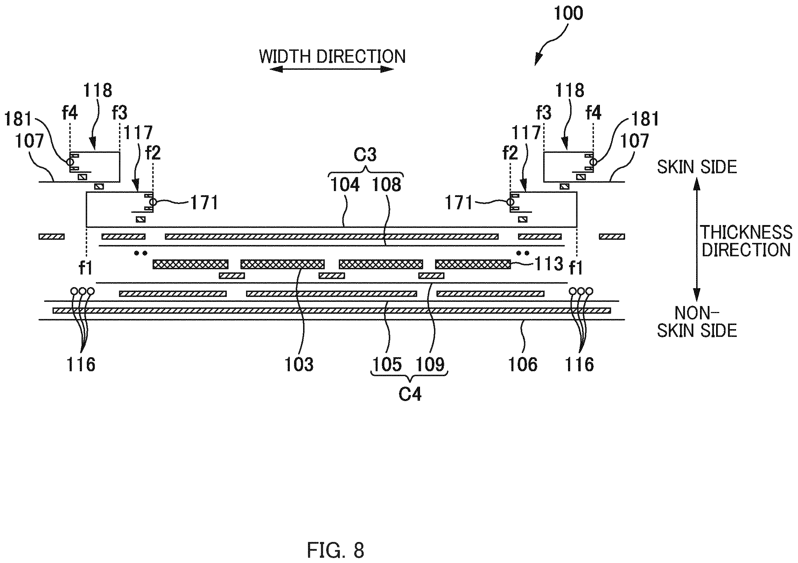

FIG. 7 is a schematic plan view of the disposable diaper 100 in the unfolded and stretched state, which is an absorbent article according to the second embodiment, as viewed from the skin surface side. FIG. 8 is a cross-sectional view taken along line A-A shown in FIG. 7.

Similarly to the first embodiment, the diaper 100 has the lengthwise direction, the width direction, and the thickness direction the unfolded and stretched state (FIG. 7). In addition, the diaper 100 is divided into three regions in the lengthwise direction, and includes: a front waist portion 101A that is applied to the wearer's stomach side; a back waist portion 101C that is applied to the wearer's back side; and a crotch portion 101B that is located between front waist portion 101A and the back waist portion 101C in the lengthwise direction. Further, as shown in FIG. 8, the direction in which the constituent members of the diaper 100 are overlaid on each other is referred to as the thickness direction. In the thickness direction, the side that is in contact with the wearer is called the skin side, and the opposite side is called the non-skin side. Note that the unfolded state and the stretched state of the diaper 100 are the same as the definitions of the unfolded state and the stretched state in the first embodiment.

In addition, as shown in FIG. 2, the diaper 100 includes: an absorbent core 103; a top-surface sheet 104 provided on the skin side in the thickness direction with respect to the absorbent core 103; a back-surface sheet 105 and an exterior sheet 106 that are provided on the non-skin side in the thickness direction with respect to the absorbent core 103, and a pair of side sheets 107.

The top-surface sheet 104 only needs to be a liquid-permeable sheet, and examples thereof include an air-through nonwoven fabric sheet, a spunbond nonwoven fabric sheet and the like. The back-surface sheet 105 only needs to be a liquid-impermeable sheet, and examples thereof include a synthetic resin film and a hydrophobic SMS nonwoven fabric sheet and the like. Additionally, the top-surface sheet 104 and the back-surface sheet 105 contain thermoplastic resin (polyethylene (PE), polypropylene (PP), and the like).

The pair of side sheets 107 each are a sheet (e.g., nonwoven fabric sheet, and the like) located on the skin side of the top-surface sheet 104 and extending outward in the width direction from both side portions in the width direction. The exterior sheet 106 is a liquid-impermeable sheet located on the non-skin side in the thickness direction with respect to the back-surface sheet 105, and has a shape (that is, the external shape of the diaper 101) in which the center portion (crotch portion 101B) in the lengthwise direction is narrowed inward in the width direction.

For example, the absorbent core 103 can be obtained by molding liquid-absorbent fibers (e.g., pulp) containing SAP (superabsorbent polymer) into a predetermined shape. In addition, in the present embodiment, a liquid-permeable skin-side core-wrapping sheet 108 is arranged adjacent to the skin side of the absorbent core 103, and a liquid-permeable non-skin-side core-wrapping sheet 109 is arranged adjacent to the non-skin side of the absorbent core 103. Further, the skin-side core-wrapping sheet 108 and the non-skin-side core-wrapping sheet 109 contain thermoplastic resin in addition to pulp fiber as the liquid-absorbent fiber. Examples of the thermoplastic resin include the polyethylene (PE) and the polypropylene (PP). The details of the skin-side core-wrapping sheet 108 and the non-skin-side core-wrapping sheet 109 will be described later.

The diaper 100 also includes flap portions 110 extending outward in the width direction from the back waist portion 101C, as shown in FIG. 1. The flap portions 110 each include a base sheet 111, and a first fastening member 112 and a second fastening member 113 that are joined to the base sheet 111. The base sheet 111 is joined between the side sheet 107 and the exterior sheet 106. The first fastening member 112 and the second fastening member 113 are arranged spacing in the lengthwise direction from each other, and are configured to be able to fasten to a target portion 114 when putting on the diaper 100, the target portion 114 being provided in the front waist portion 101A. The first fastening member 112 and the second fastening member 113 can be a hook material, for example. Note that a configuration may be acceptable in which one fastening member is provided on each of the left and right sides. Moreover, a configuration may be acceptable in which the diaper 100 does not include the base sheet 111 of the flap portion 110, but the side sheet 107 and the exterior sheet 106 extend outward in the width direction, and the fastening members are joined onto the side sheet 107.

The diaper 100 has leg elastic members 116 that stretches and contracts in the lengthwise direction on both side portions in the width direction (see FIG. 8). Three leg elastic members 116 are provided on each side in the width direction with spacing from each other. More specifically, the leg elastic member 116 that is located farthest inside in the width direction is arranged between the top-surface sheet 104 and the back-surface sheet 105. The remaining two leg elastic members 116 are arranged between the side sheet 107 and the back-surface sheet 105. Note that the number of the leg elastic members 116 is not particularly limited.

Moreover, the diaper 100 has inner leak-proof gathers 117 and outer leak-proof gathers 118. The inner leak-proof gathers 117 and the outer leak-proof gathers 118 are configured to be able to rise up on the side of the wearer's skin surface, and are provided in pair on the left and right sides. The inner leak-proof gathers 117 are so-called inward-folding gathers, and the outer leak-proof gathers 118 are so-called outward-folding gathers. The inner leak-proof gathers 117 are each provided inside the corresponding outer leak-proof gather 118 and leg elastic members 116 in the width direction. Note that it is sufficient that at least a part of the inner leak-proof gather 117 is located inside the corresponding outer leak-proof gather 118 in the width direction. Moreover, the diaper 100 only needs to have at least the outer leak-proof gathers 118, and does not need to have the inner leak-proof gathers 117.

The inner leak-proof gather 117 is formed by folding the top-surface sheet 104, as shown in FIG. 8. Specifically, the inner leak-proof gather 117 is formed as follow: the top-surface sheet 104 is folded inward from the outside in the width direction and toward the skin side at a folding line f1 shown in FIG. 2, and further the top-surface sheet 104 is folded back outward from the inside in the width direction and toward the non-skin side at a folding line f2, and an inner leak-proof elastic member 171 that stretches and contracts in the lengthwise direction is provided in the folded-back portion. Examples of the inner leak-proof elastic member 171 include elastic strings.

The outer leak-proof gather 118 is formed by folding the side sheet 107, as shown in FIG. 2. Specifically, the outer leak-proof gather 118 is formed as follow: the side sheet 107 is folded outward from the inside in the width direction and toward the skin side at the folding line f3 shown in FIG. 2, and further the side sheet 107 is folded back inward from the outside in the width direction and toward the non-skin side at the folding line f4, and an outer leak-proof elastic member 181 that stretches and contracts in the lengthwise direction is provided in the folded-back portion. Examples of the outer leak-proof elastic member 181 include elastic strings. The inner leak-proof gathers 117 and the outer leak-proof gathers 118 can suppress lateral leakage of excrement.

In the back waist portion 101C of the diaper 100, a waist gather 122 formed by a plurality of waist elastic members 121 is arranged in the end in the lengthwise direction and in the center in the width direction. Due to the waist gather 122, the diaper 100 deforms to fit the shape of the wearer's waist when putting on, and also is suppressed diaper 100 from shifting.

### Core-wrapping sheet, separate sheet and composite sheet in second embodiment

Similarly to the first embodiment, the skin-side core-wrapping sheet 108 and the non-skin-side core-wrapping sheet 109 in the second embodiment contain recycled pulp that is obtained by recycling used disposable absorbent article (e.g., used disposable diapers and absorbent pads). The diaper 100 includes a composite sheet in which the skin-side core-wrapping sheet 108 and a separate sheet that is adjacent to the skin-side core-wrapping sheet 108 in the thickness direction of the diaper 100 are joined. In the present embodiment, the separate sheet for the skin-side core-wrapping sheet 108 is the top-surface sheet 104, and the skin-side core-wrapping sheet 108 and the top-surface sheet 104 are joined to form a skin-side composite sheet C3, which is a composite sheet. The skin-side composite sheet C3 is placed on the skin side of the absorbent core 103 in the thickness direction. Moreover, the diaper 100 includes a composite sheet in which the non-skin-side core-wrapping sheet 109 and a separate sheet that is adjacent to the non-skin side core-wrapping sheet 109 in the thickness direction of the diaper 100 are joined. The separate sheet for the non-skin-side core-wrapping sheet 109 is the back-surface sheet 105, and the non-skin-side core-wrapping sheet 109 and the back-surface sheet 105 are joined to form a non-skin-side composite sheet C4, which is a composite sheet. The non-skin-side composite sheet C4 is placed on the non-skin side of the absorbent core 103 in the thickness direction.

In addition, the effect exhibited by the first composite sheet C1 in the first embodiment described above is the same in the skin-side composite sheet C3 in the second embodiment, and the effect exhibited by the second composite sheet C2 in the first embodiment is the same in the non-skin-side composite sheet C4 in the second embodiment. Further, in the following description, common functions and effects will be explained.

In the present embodiment, the strength of the materials is also required so that the skin-side core-wrapping sheet 108 and the non-skin-side core-wrapping sheet 109, which contain the recycled pulp, are not damaged while using the diaper 100. Therefore, in order to confirm the material strength of the skin-side core-wrapping sheet 108 in the skin-side composite sheet C3 in which the skin-side core-wrapping sheet 108 and the top-surface sheet 104 (separate sheets) are joined, a test was conducted using the following method.

### Evaluation method

The followings are prepared as materials for evaluation:
A) Recycled tissue as the skin-side core-wrapping sheet 108 containing 15 wt% of the recycled pulp;
B) As an example of the top-surface sheet 104 (separate sheet), polypropylene spunbond nonwoven fabric sheet (PPSB);
C) As an example of the top-surface sheet 104 (separate sheet), air-through nonwoven fabric sheet;
D) As an example of the skin-side composite sheet C3, composite sheet A made by laminating the sheets a) and b) described above with a hot-melt adhesive (application amount of the adhesive is 4 g/m2 and is applied in a spiral pattern); and
E) As an example of the skin-side composite sheet C3, composite sheet B made by laminating the sheets a) and c) described above with a hot-melt adhesive (application amount of the adhesive is 4 g/m2 and is applied in a spiral pattern). Each of the sheets A) to E) described above was cut out to a size of 25 mm x 100 mm, and samples of each sheet were produced. Note that, in order to measure the strength of each sheet in a fiber orientation direction, the length of the sheet in the fiber orientation direction was set to 100 mm. In the case where the material is a nonwoven fabric sheet, the constituent fibers are arranged in the direction in which the nonwoven fabric web is conveyed (MD direction) when manufacturing the nonwoven fabric web, for example, and accordingly the fiber orientation direction means the direction along which the fibers of such material extend. The tensile strength factor of the material (sheet) in the orientation direction of the constituent fibers (MD direction of web) is originally higher than the tensile strength factor in the direction perpendicular to the orientation direction of the constituent fibers (CD direction of web). That is, the fiber orientation direction is also the direction, of the lengthwise direction and the width direction of the diaper 100, whichever has the higher strength of the material. In the present embodiment, in the unfolded state shown in FIG. 7, the fiber orientation direction is the lengthwise direction of the diaper 100, and the direction perpendicular to the orientation direction of the constituent fibers is the width direction.

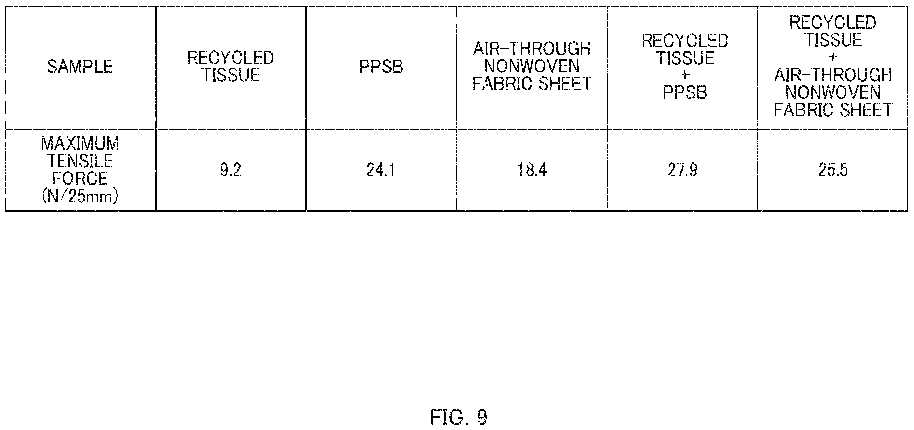

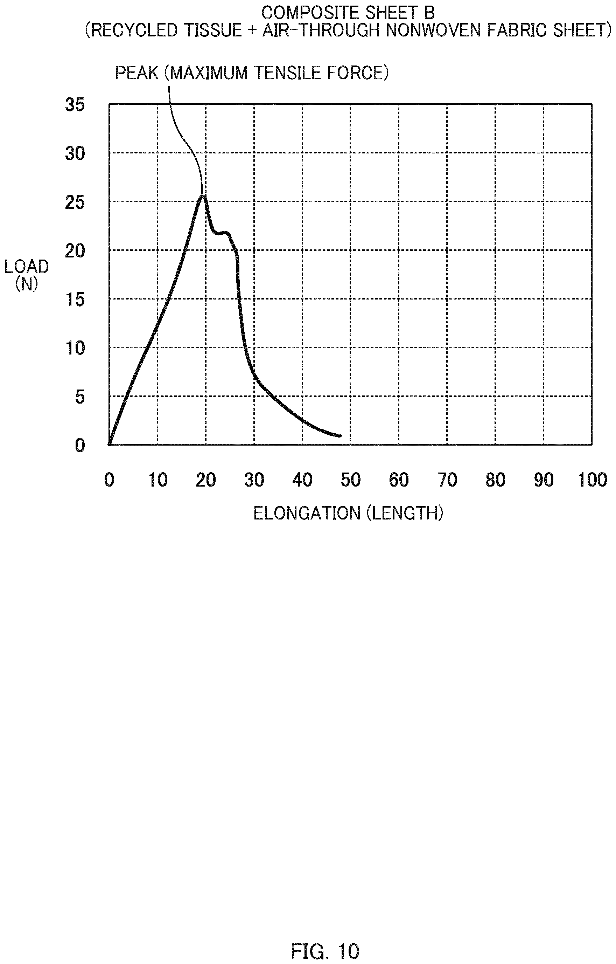

Then, using a tensile tester (autograph tensile tester AGS-1KNG manufactured by Shimadzu Corporation, and the like), the sample was pulled in the fiber orientation direction at a speed of 100 mm/min to measure the tensile strength. The same measurement was performed five times for each sample to calculate the respective maximum tensile force, and the average thereof was taken as the value of the maximum tensile force (tensile strength) for each material a) to e). FIG. 9 is a table showing the evaluation results of the tensile strength of the sheets. Note that the width of the sheets is set to 25 mm as a standard, and therefore the denominator of the unit of maximum tensile force is 25 mm. Further, FIG. 10 shows an example of a load-elongation diagram in a tensile test of the above-mentioned composite sheet B. Among five measurements made using samples of the composite sheet B, as an example, the diagram in FIG. 10 shows the result of the sample whose maximum tensile force is close to the average value. Note that the peak (maximum point) shown in the diagram of FIG. 10 is the maximum tensile force, and this peak is the point at which the sheet begins to break. In reality, the breakage is completed after the point of maximum tensile force.

Referring to FIG. 9, the maximum tensile force of the recycled tissue alone, which is the sheet A), was 9.2 N/25mm. On the other hand, the maximum tensile force of the composite sheet A made by joining the recycled tissue and PPSB, which is the sheet D), was 27.9 N/25mm, and the maximum tensile force of composite sheet B made by joining the recycled tissue and the air-through nonwoven fabric sheet, which is the sheet E) was 25.5 N/25mm. That is, the strength increased. The maximum tensile force of the composite sheet A is higher than the maximum tensile force (24.1 N/25mm) of PPSB alone, which is the sheet B), that is, its separate sheet. Also, the maximum tensile force of the composite sheet B is higher than the maximum tensile force (18.4 N/25mm) of the air-through nonwoven fabric alone, which is the sheet C), that is, its separate sheet.

Here, the maximum tensile force per unit width (25 mm) of each sheet is the tensile force per unit width at which the sheet begins to break, i.e., the tensile force per unit width at which the recycled tissue in each of the composite sheets A and B starts to break. Therefore, from the above-described test, concerning the tensile force per unit width at the timing when the skin-side core-wrapping sheet 108 of the skin-side composite sheet C3 starts to break in the case of pulling the skin-side composite sheet C3 in the fiber orientation direction of the skin-side core-wrapping sheet 108, concerning the tensile force per unit width at the timing when the separate sheet (top-surface sheet 104) starts to break in the case of pulling the separate sheet alone in the fiber orientation direction, it can be said that the former tensile force is higher than the latter tensile force. In other words, by joining the skin-side core-wrapping sheet 108 and the top-surface sheet 104, the strength of the skin-side core-wrapping sheet 108 becomes a strength that the skin-side core-wrapping sheet 108 do not break unless there is applied a force greater than the tensile force at the timing when the top-surface sheet 104 alone starts to break. Since such a skin-side core-wrapping sheet 108 having such strength is used for the diaper 100, even if the wearer uses it for a long time, it is possible to reduce a risk of the skin-side core-wrapping sheet 108 being damaged.

The above results are the effects obtained when using the skin-side core-wrapping sheet 108 in which the content of the recycled pulp is 15 wt%. It is more preferable that the content of the recycled pulp in the skin-side core-wrapping sheet 108 is less than 15 wt%. By making the content be less than 15 wt%, the skin-side core-wrapping sheet 108 is able to maintain the above-mentioned strength. By using such a skin-side core-wrapping sheet 108 for the diaper 100, it is possible to reduce the risk of damage to the skin-side core-wrapping sheet 108 even when used for a long time.

Furthermore, the following can be said about the strength of the non-skin-side core-wrapping sheet 109 in the non-skin-side composite sheet C4. As mentioned above, in the present embodiment, the separate sheet of the skin-side composite sheet C3 (top-surface sheet 104) is a liquid-permeable sheet member, and the separate sheet of the non-skin-side composite sheet C4 (back-surface sheet 105) is a liquid-impermeable sheet member. Therefore, concerning the tensile force per unit width at the timing when the non-skin-side core-wrapping sheet 109 starts to break in the non-skin-side composite sheet C4 in the case of pulling the non-skin-side composite sheet C4 in the fiber orientation direction of the non-skin-side core-wrapping sheet 109, concerning the tensile force per unit width at the timing when the skin-side core-wrapping sheet 108 starts to break in the skin-side composite sheet C3 in the case of pulling the skin-side composite sheet C3 in the fiber orientation direction of the skin-side core-wrapping sheet 108, the former tensile force is higher than the latter tensile force. In other words, the tensile force at the timing when the skin-side core-wrapping sheet 108 and the non-skin-side core-wrapping sheet 109 in each composite sheet starts to break increases as the tensile force at the timing when the separate sheet to be joined starts to break increases. The non-skin-side composite sheet C4 is joined to the liquid-impermeable separate sheet (back-surface sheet 105) such as a synthetic resin film, and therefore the non-skin-side core-wrapping sheet 109 of the non-skin-side composite sheet C4 has a higher tensile force at the timing of starting of breakage than the skin-side core-wrapping sheet 108 of the skin-side composite sheet C3. That is, the non-skin-side core-wrapping sheet 109 in the non-skin-side composite sheet C4 is less likely to break than the skin-side core-wrapping sheet 108 in the skin-side composite sheet C3. Therefore, compared to the case where the tensile force per unit width at the timing when the non-skin-side core-wrapping sheet 109 starts to break in the non-skin-side composite sheet C4 is lower than the tensile force per unit width at the timing when the skin-side core-wrapping sheet 108 starts to break in the skin-side composite sheet C3, this makes it possible to further improve leakage prevention effect in the case where the tensile force per unit width at the timing when the non-skin-side core-wrapping sheet 109 starts to break in the non-skin-side composite sheet C4 is higher than the tensile force per unit width at the timing when the skin-side core-wrapping sheet 108 starts to break in the skin-side composite sheet C3.

In addition, in the present embodiment, the skin-side core-wrapping sheet 108 and the top-surface sheet 410 are joined to form the skin-side composite sheet C3, which is a composite sheet. But as a modified example of the diaper 100, it is also conceivable that another sheet material such as an auxiliary sheet is arranged between the skin-side core-wrapping sheet 108 and the top-surface sheet 104, for example. However, the composite sheet in the present embodiment is formed by joining the skin-side core-wrapping sheet 108 with the separate sheet that can prevent the contents of the absorbent core 103 from leaking out due to damage, and therefore it is preferable that the separate sheet is not an auxiliary sheet or the like, but is the top-surface sheet 104.

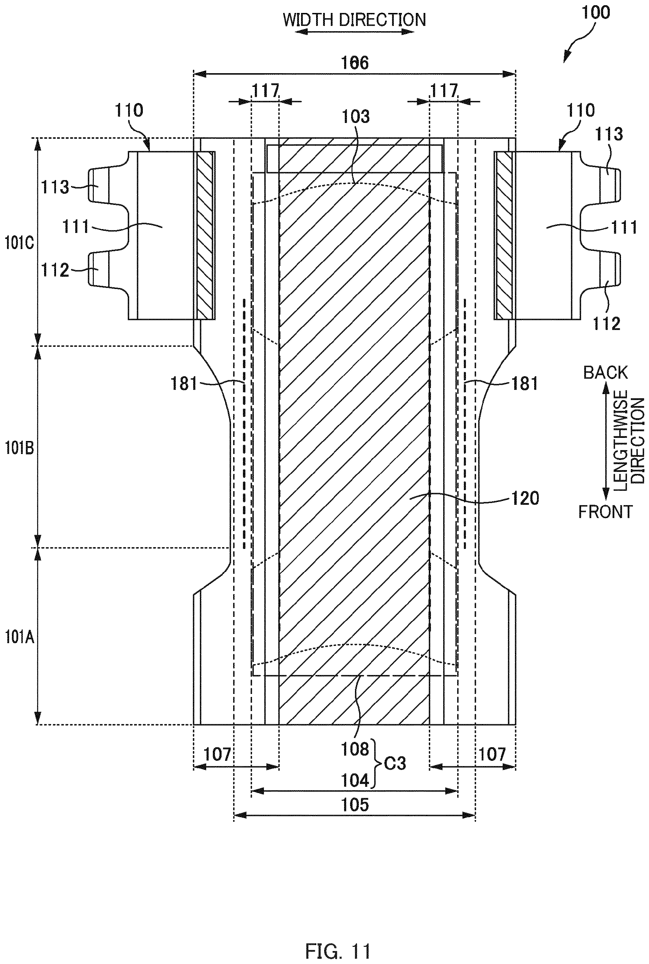

FIG. 11 is a plan view showing the application range of the adhesive which joins the top-surface sheet 104 and the skin-side core-wrapping sheet 108. In FIG. 11, the application region 120 of the adhesive (the downward-left hatched portion in the center of the width direction) is shown with the top-surface sheet 104 and the skin-side core-wrapping sheet 108 seeing through. As shown in the figure, in the skin-side composite sheet C3 formed by the top-surface sheet 104 and the skin-side core-wrapping sheet 108, the skin-side core-wrapping sheet 108 and the top-surface sheet 104 are joined with a hot-melt adhesive applied in a planar manner. By joining with hot-melt adhesive, it is possible to make the skin-side core-wrapping sheet 108 of the skin-side composite sheet C3 further less likely to break even when the skin-side core-wrapping sheet 108 continues is infiltrated with liquid such as excreted fluid.

In addition, as shown in FIG. 11, the hot-melt adhesive is applied in a planar manner in the application region 120, that is, the hot-melt adhesive is applied without any gaps, and this makes it possible to increase the strength of the skin-side core-wrapping sheet 108.

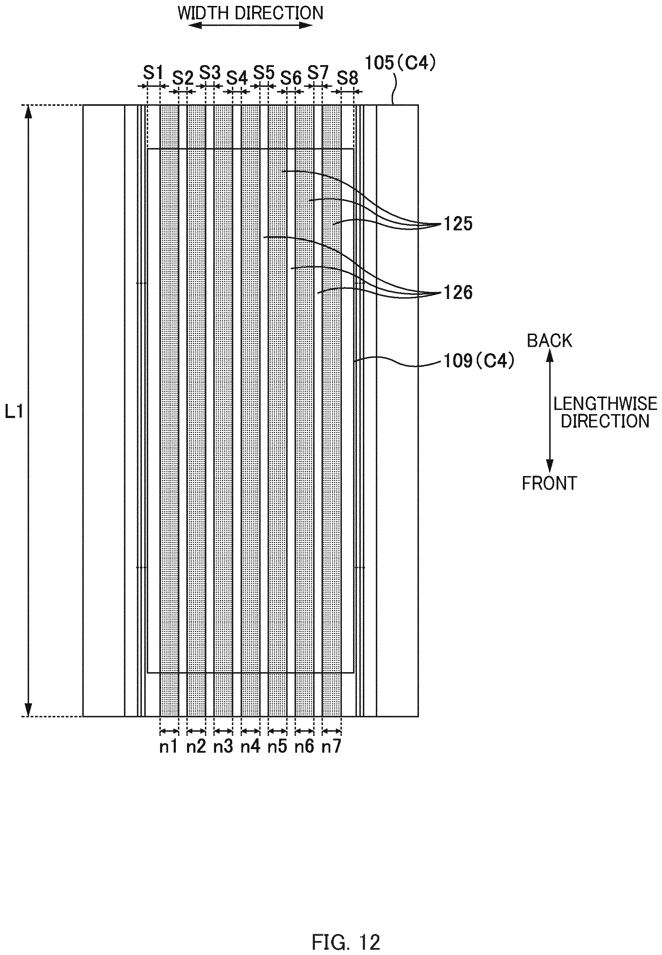

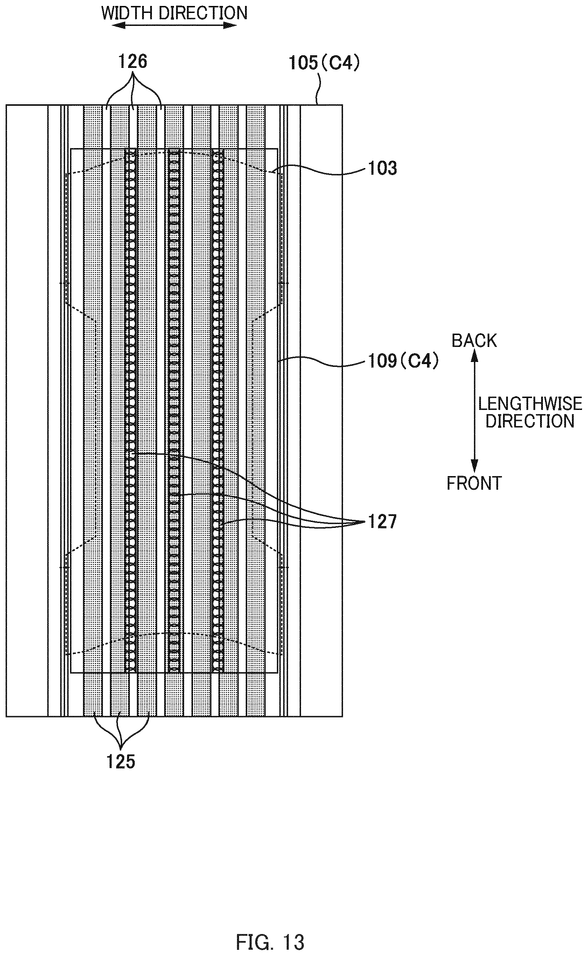

FIG. 12 is a plan view showing the application range of the adhesive which joins the non-skin-side core-wrapping sheet 109 and the back-surface sheet 105. FIG. 13 is a plan view further showing the adhesive with which the absorbent core 103 and the non-skin-side core-wrapping sheet 109 are joined, onto the plan view of FIG. 12. In both of FIGS. 12 and 13, the diaper 100 that is in the unfolded state is shown in plan view, and for convenience of explanation, the constituent materials are limited. In FIG. 12, the application range of the adhesive is shown with the non-skin-side core-wrapping sheet 109 and the back-surface sheet 105 seeing through. Also, in FIG. 13, the application range of the adhesive is shown with the non-skin-side core-wrapping sheet 109, the back-surface sheet 105, and the absorbent core 103 seeing through.

In the present embodiment, as shown in FIG. 12, in the non-skin-side composite sheet C4 composed of the non-skin-side core-wrapping sheet 109 and the back-surface sheet 105, the non-skin-side core-wrapping sheet 109 and the back-surface sheet 5 are joined with the hot-melt adhesive. Specifically, the non-skin-side composite sheet C4 has a plurality of joining regions 125 where the non-skin-side core-wrapping sheet 109 and the back-surface sheet 105 are joined with the hot-melt adhesive. In the present embodiment, seven joining regions 25 are provided spacing in the width direction and extending along the lengthwise direction, but the number of the joining regions 25 is not limited to this. Examples of the application pattern of the hot-melt adhesive include an Ω pattern, a spiral pattern, and a solid coating pattern in which the adhesive is applied to the application target region without any gaps, but the present invention is not limited to these. In addition, in the case where the joining region 25 is formed by the hot-melt adhesive being applied, for example, in a circular application pattern, the width of the joining region 25 (later-described n1, n2, and the like) is not the width of the line which draws the circular pattern, but the diameter of the circular pattern. Also, the back-surface sheet 105 is made of a liquid-impermeable sheet member (synthetic resin film, and the like), and therefore by joining it with the hot-melt adhesive in this way, the non-skin-side core-wrapping sheet 109 in the non-skin-side composite sheet C4 is made further less likely to break, making it possible to reduce a risk of leakage of the diaper 100 from the non-skin side.

Further, as shown in FIG. 12, the non-skin-side composite sheet C4 has non-joining regions 126 where the non-skin-side core-wrapping sheet 109 and the back-surface sheet 105 are not joined with the hot-melt adhesive. Similarly, in the skin-side composite sheet C3, the region excluding the application region 120 in the skin-side core-wrapping sheet 108 shown in FIG. 11 becomes a non-joining region. For example, if the hot-melt adhesive is applied completely without any gaps to the entire region where the non-skin-side core-wrapping sheet 109 and the back-surface sheet 105 overlap, there is a risk that the non-skin-side composite sheet C4 becomes stiff. However, by having the non-joining regions 126, the softness can be maintained and a balance between strength and hardness can be achieved.

Here, as shown in FIG. 12, letting the lengths of the joining regions 125 in the width direction be n1, n2, ... n7, letting the lengths of the non-joining regions 126 in the width direction be S1, S2, ... S8, in the present embodiment, the total length of the joining regions 125 in the width direction in the non-skin-side composite sheet C4 (n1 + n2 + n3 + n4 + n5 + n6 + n7) is longer than the total length of the non-joining regions 126 in the width direction (S1 + S2 + S3 + S4 + S5 + S6 + S7 + S8). In other words, in the non-skin-side composite sheet C4, the region that is fixed with the hot-melt adhesive is larger. By increasing the number of non-joining regions 25 in this way, it is possible to improve the strength of the non-skin-side composite sheet C4, making the non-skin-side core-wrapping sheet 109 of the non-skin-side composite sheet C4 further less likely to break.

In addition, the length L1 of a portion in which hot-melt adhesive is provided continuously in the lengthwise direction of the non-skin-side composite sheet C4 (i.e. the length of the joining regions 125 in the lengthwise direction) is longer than the length of a portion in which hot-melt adhesive is provided continuously in the width direction (any one of the lengths n1 to n7) . For the non-skin-side core-wrapping sheet 109, the tensile strength in the lengthwise direction, which is the fiber orientation direction, is higher than the tensile strength in the width direction. Since the hot-melt adhesive is provided elongated continuously in the lengthwise direction, which is the fiber orientation direction, it is possible to make the non-skin-side core-wrapping sheet 109 further less likely to break.

Further, as shown in FIG. 11, the joining region where the skin-side core-wrapping sheet 108 and the top-surface sheet 104 are joined with the hot-melt adhesive (application region 120) is provided over the entire range of the skin-side core-wrapping sheet 108 in the lengthwise direction. Similarly, as shown in FIG. 6, the joining regions 125 where the non-skin-side core-wrapping sheet 109 and the back-surface sheet 105 are joined with the hot-melt adhesive are each provided over the entire range of the non-skin-side core-wrapping sheet 109 in the lengthwise direction. Note that it is sufficient that at least a part of the joining region 125 is provided over the entire range of the non-skin-side core-wrapping sheet 109 in the lengthwise direction. In the lengthwise direction, in which the tensile strength is high, since the application regions 120 (joining regions 125) of the hot-melt adhesive exist the entire range of the skin-side core-wrapping sheet 108 (non-skin-side core-wrapping sheet 109) in the lengthwise direction, it can make the skin-side core-wrapping sheet 108 (non-skin-side core-wrapping sheet 109) further less likely to break.

In the present embodiment, as mentioned above, the skin-side core-wrapping sheet 108 of the skin-side composite sheets C3 placed on the skin side in the thickness direction of the absorbent core 103, is placed adjacent to the absorbent core 103 on the skin side. Further, the non-skin-side core-wrapping sheet 109, which is a recycled sheet of the non-skin-side composite sheet C4 placed on the non-skin side in the thickness direction of the absorbent core 103, is placed adjacent to the absorbent core 103 on the non-skin side. The absorbent core 3 swells when it absorbs liquid, and therefore if the core-wrapping sheet is provided so as to wrap around the absorbent core 103, the swelling of the absorbent core 103 makes a force be applied to the core-wrapping sheet, causing a risk that the core-wrapping sheet on the side that wraps the absorbent core 103 breaks. In the present embodiment, by arranging the skin-side core-wrapping sheet 108 and the non-skin-side core-wrapping sheet 109 individually on the skin side and the non-skin side, it is possible to reduce the burden on the core-wrapping sheet and to reduce the risk of breakage.

As shown in FIG. 13, the absorbent core 103 and the non-skin-side core-wrapping sheet 109 have core joining regions 27 where they are joined to each other with a hot-melt adhesive. In the present embodiment, three core joining regions 127 are provided spacing in the width direction and extending along the lengthwise direction, but the number of the core joining regions 127 is not limited to this. The non-joining regions 126 where the non-skin-side core-wrapping sheet 109 and the back-surface sheet 105 are not joined with the hot-melt adhesive has a portion that overlaps the core joining region 127 in a plan view. There is a risk that the strength of the non-joining region 126 where the hot-melt adhesive is not applied decreases, but by having a portion that overlaps the core joining region 127, it is possible to suppress the decrease in strength of the recycled sheet of the non-skin-side composite sheet C4 (non-skin-side core-wrapping sheet 109).

### Others

The above embodiments are simply to facilitate understanding of the present disclosure and are not in any way to be construed as limiting the present disclosure. The present disclosure may variously be changed or altered without departing from its gist and encompass equivalents thereof.

In the first and second embodiments described above, the first core-wrapping sheet 38, second core-wrapping sheet 39, the skin-side core-wrapping sheet 108, and the non-skin-side core-wrapping sheet 109 contain the recycled pulp that is obtained by recycling disposable absorbent articles, but the present invention is not limited to this. For example, the first absorbent core 33a, the second absorbent core 33b, and the absorbent core 103 may contain the recycled pulp, or another sheet material constituting the absorbent article may contain the recycled pulp.

### REFERENCE SIGNS LIST

1 absorbent pad (absorbent article) (first embodiment)
33 absorbent body
33a first absorbent core
33b second absorbent core
34 top-surface sheet (separate sheet)
35 back-surface sheet (separate sheet)
36 exterior sheet
37 side sheet
38 first core-wrapping sheet
39 second core-wrapping sheet
45a slit
45b slit
46 a pair of compressed portions
48 leak-proof wall
60 leak-proof-wall elastic member
100 diaper (absorbent article) (second embodiment)
101A front waist portion
101B crotch portion
101C back waist portion
103 absorbent core
104 top-surface sheet (separate sheet)
105 back-surface sheet (separate sheet)
106 exterior sheet
107 side sheet
108 skin-side core-wrapping sheet
109 non-skin-side core-wrapping sheet
110 flap portion
111 base sheet
112 first fastening member
113 second fastening member
114 target portion
116 leg elastic member
117 inner leak-proof gather
118 outer leak-proof gather
120 application region
121 waist elastic member
122 waist gathers
125 joining region
126 non-joining region
127 core joining region
171 inner leak-proof elastic member
181 outer leak-proof elastic member
C1 first composite sheet (composite sheet)
C2 second composite sheet (composite sheet)
C3 skin-side composite sheet (composite sheet)
C4 non-skin-side composite sheet (composite sheet)

## Claims

1. An absorbent article comprising:
an absorbent core;
a core-wrapping sheet that contains recycled pulp that is obtained by recycling a disposable absorbent article; and
a separate sheet that is adjacent to the core-wrapping sheet in a thickness direction of the absorbent article,
a composite sheet is formed by joining the core-wrapping sheet with the separate sheet so that the core-wrapping sheet does not break when the absorbent article that has been immersed in 0.9% physiological saline solution for 30 minutes is hanged for 10 minutes in any of a case of being folded one time at a center in a product lengthwise direction and a case of being folded one time at a center in a product width direction.

2. The absorbent article according to claim 1, wherein
the absorbent article has
a skin-side composite sheet that is the composite sheet placed on the skin side in the thickness direction of the absorbent core, and
a non-skin-side composite sheet that is the composite sheet placed on the non-skin side in the thickness direction of the absorbent core,
the core-wrapping sheet in the skin-side composite sheet is a skin-side core-wrapping sheet that is located adjacent to the absorbent core on a skin side, and
the core-wrapping sheet in the non-skin-side composite sheet is a non-skin-side core-wrapping sheet that is located adjacent to the absorbent core on a non-skin side.

3. The absorbent article according to claim 1 or 2, wherein
the absorbent core contains superabsorbent polymer, and
a mass ratio of the superabsorbent polymer in the absorbent core is 28% or less

4. The absorbent article according to claim 1 or 3, wherein
the absorbent article includes
a first absorbent core that contains at least a superabsorbent polymer, and
a second absorbent core that contains at least a superabsorbent polymer,
a mass ratio of the superabsorbent polymer contained in the first absorbent core is greater than a mass ratio of the superabsorbent polymer contained in the second absorbent core,
a first core-wrapping sheet that is arranged so as not to wrap the first absorbent core and that is the core-wrapping sheet is provided, and
a second core-wrapping sheet that wraps the second absorbent core and that is the core-wrapping sheet is provided.

5. The absorbent article according to any one of claims 1 to 4, wherein
the core-wrapping sheet contains a thermoplastic resin.

6. The absorbent article according to claim 5, wherein
the separate sheet contains a thermoplastic resin, and
the core-wrapping sheet and the separate sheet are thermally-fused.

7. The absorbent article according to claim 6, wherein
the thermoplastic resin of the core-wrapping sheet and the thermoplastic resin of the separate sheet are an identical type of thermoplastic resin.

8. The absorbent article according to claim 4, wherein
the core-wrapping sheet contains a thermoplastic resin,
at least a part of the core-wrapping sheet has an overlapping region where portions of the core-wrapping sheet overlap each other, in a plan view of the absorbent article that is in the unfolded state, and
in the overlapping region, the portions of the core-wrapping sheet are thermally-fused to each other.

9. The absorbent article according to any one of claims 1 to 4, wherein
a content of the recycled pulp in the core-wrapping sheet being less than 15 wt%, and
a tensile force per unit width at a timing when the core-wrapping sheet starts to break in the composite sheet if the composite sheet is pulled in a fiber orientation direction of the core-wrapping sheet
is higher than
a tensile force per unit width at a timing when the separate sheet starts to break if the separate sheet alone is pulled in the fiber orientation direction.

10. The absorbent article according to any one of claims 1 to 9, wherein
in the composite sheet, the core-wrapping sheet and the separate sheet are joined with a hot-melt adhesive.

11. The absorbent article according to claim 10, wherein
the hot-melt adhesive is applied in a planar manner to at least a part.

12. The absorbent article according to claim 10, wherein
the composite sheet has a non-joined region where the core-wrapping sheet and the separate sheet are not joined with the hot-melt adhesive.

13. The absorbent article according to claim 10 or 11, wherein
at least a part of a joining region where the core-wrapping sheet and the separate sheet are joined with the hot-melt adhesive is provided over an entire range of the core-wrapping sheet in the fiber orientation direction.

14. The absorbent article according to claim 2, wherein
the separate sheet of the non-skin-side composite sheet is a liquid-impermeable sheet member, and
in the non-skin-side composite sheet,
the non-skin-side core-wrapping sheet and the separate sheet are joined with a hot-melt adhesive.

15. The absorbent article according to claim 2 or 14, wherein
the separate sheet of the non-skin-side composite sheet is located adjacent to the non-skin-side core-wrapping sheet on the non-skin side,
the non-skin-side composite sheet has a non-joined region where the non-skin-side core-wrapping sheet and the separate sheet are not joined with a hot-melt adhesive,
the absorbent core and the non-skin-side core-wrapping sheet have a core joining region where the absorbent core and the non-skin-side core-wrapping sheet are joined to each other with the hot-melt adhesive, and
the non-joining region has a portion that overlaps the core joining region in a plan view of the absorbent article that is in an unfolded state.

16. The absorbent article according to any one of claims 2, 14, and 15, wherein
the separate sheet of the skin-side composite sheet is a liquid-permeable sheet member,
the separate sheet of the non-skin-side composite sheet is a liquid-impermeable sheet member, and
a tensile force per unit width at a timing when the core-wrapping sheet starts to break in the non-skin-side composite sheet if the non-skin-side composite sheet is pulled in a fiber orientation direction of the core-wrapping sheet
is higher than
a tensile force per unit width at a timing when the core-wrapping sheet starts to break in the skin-side composite sheet if the skin-side composite sheet is pulled in the fiber orientation direction of the core-wrapping sheet.
